(19) Europäisches Patentamt
European Patent Office
Office européen des brevets

(11) **EP 3 285 854 B1**

(12) **EUROPÄISCHE PATENTSCHRIFT**

(45) Veröffentlichungstag und Bekanntmachung des Hinweises auf die Patenterteilung:
**24.04.2019 Patentblatt 2019/17**

(21) Anmeldenummer: **16731596.9**

(22) Anmeldetag: **22.06.2016**

(51) Int Cl.:
*A61N 1/05* *(2006.01)*     *A61N 1/36* *(2006.01)*
*A61N 5/06* *(2006.01)*

(86) Internationale Anmeldenummer:
**PCT/EP2016/064471**

(87) Internationale Veröffentlichungsnummer:
**WO 2016/207246 (29.12.2016 Gazette 2016/52)**

(54) **VORRICHTUNG ZUR EFFEKTIVEN INVASIVEN ZWEI-STUFEN-NEUROSTIMULATION**

DEVICE FOR EFFECTIVE INVASIVE TWO-STAGE NEUROSTIMULATION

DISPOSITIF DE STIMULATION NEUROLOGIQUE INVASIVE EFFICACE À DEUX NIVEAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.06.2015 DE 102015109988**

(43) Veröffentlichungstag der Anmeldung:
**28.02.2018 Patentblatt 2018/09**

(73) Patentinhaber: **Forschungszentrum Jülich GmbH 52425 Jülich (DE)**

(72) Erfinder:
• **TASS, Peter Alexander**
  **83684 Tegernsee (DE)**
• **ZEITLER, Magteld**
  **6581 JL Malden (NL)**

(74) Vertreter: **Vossius & Partner Patentanwälte Rechtsanwälte mbB Siebertstrasse 3 81675 München (DE)**

(56) Entgegenhaltungen:
**DE-A1-102008 052 078     DE-A1-102009 015 723
DE-A1-102010 016 404     DE-A1-102010 016 461**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur effektiven invasiven Zwei-Stufen-Neurostimulation mittels variierender Reizsequenzen.

Bei Patienten mit neurologischen oder psychiatrischen Erkrankungen, z. B. Morbus Parkinson, essentiellem Tremor, Epilepsie, Funktionsstörungen nach Schlaganfall, Dystonie oder Zwangserkrankungen, sind Nervenzellverbände in umschriebenen Bereichen des Gehirns, z. B. des Thalamus und der Basalganglien krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Zur Behandlung von medikamentös nicht hinreichend behandelbaren Parkinsonpatienten wird die tiefe Hirnstimulation eingesetzt. Hierbei werden Tiefenelektroden in speziellen Hirngebieten, z. B. im Nucleus subthalamicus, implantiert. Zur Linderung der Symptome wird über die Tiefenelektroden eine elektrische Reizung durchgeführt. Bei der Standard-Hochfrequenz-Stimulation zur Behandlung der Parkinsonschen Erkrankung wird eine sogenannte Hochfrequenz-Dauerreizung bei Frequenzen von über 100 Hz durchgeführt. Diese Behandlungsart hat keine lang anhaltenden therapeutischen Effekte (vgl. P. Temperli, J. Ghika, J.-G. Villemure, P. Burkhard, J. Bogousslavsky, and F. Vingerhoets: How do parkinsonian signs return after discontinuation of subthalamic DBS? Neurology 60, 78 (2003)). Mit weniger Reizstrom kommt die "Coordinated Reset"-Stimulation (CR-Stimulation) aus, die obendrein lang anhaltende therapeutische Effekte haben kann (P. A. Tass, L. Qin, C. Hauptmann, S. Doveros, E. Bezard, T. Boraud, W. G. Meissner: Coordinated reset neuromodulation has sustained after-effects in parkinsonian monkeys. Annals of Neurology 72, 816-820 (2012); I. Adamchic, C. Hauptmann, U. B. Barnikol, N. Pawelcyk, O.V. Popovych, T. Barnikol, A. Silchenko, J. Volkmann, G. Deuschl, W. Meissner, M. Maarouf, V. Sturm, H.-J. Freund, P. A. Tass: Coordinated Reset Has Lasting Aftereffects in Patients with Parkinson's Disease. Movement Disorders 29, 1679 (2014)).

Bei anderen Erkrankungen, z. B. bei medikamentös nicht ausreichend behandelbaren Epilepsien, werden neben Tiefenelektroden auch andere, z. B. epikortikale oder epidurale Elektroden, implantiert. Bei weiteren Erkrankungen, z. B. chronischen Schmerz-Syndromen, ist es üblich, nicht nur mittels Tiefenelektroden im Gehirn, sondern auch mittels z. B. epiduralen Elektroden das Rückenmark zu reizen. Anders als die CR-Stimulation haben die meisten anderen Stimulationsarten keine lang anhaltenden therapeutischen Effekte.

Therapeutische Effekte können auch durch direkte Stimulation des Hirngewebes bzw. Rückenmarks mit Licht, z. B. über implantierte Lichtleiter, erzielt werden. Hierbei können auch unterschiedliche raum-zeitliche Stimulationsmuster, wie z. B. die CR-Stimulation, zur Anwendung kommen.

Obgleich die tiefe Hirnstimulation mittels invasiver CR-Stimulation lang anhaltende therapeutische Effekt ermöglicht, hat dieser Ansatz relevante Limitationen. Die CR-Stimulation kann Nebenwirkungen hervorrufen, z. B. durch die nicht gewünschte Reizung von benachbarten Strukturen infolge der Ausbreitung von Reizströmen oder durch die aus anatomischen Gründen schwer vermeidbare gleichzeitige Reizung z. B. von Faserbahnen und/oder Fasern, welche in der Nachbarschaft des Zielgebiets oder sogar durch das Zielgebiet verlaufen. Derartige Situationen ergeben sich z. B. durch die charakteristische enge anatomische Nachbarschaft von bei der Elektrodenimplantation anvisiertem Zielpunkt und anderen anatomischen Strukturen, deren Reizung zu Nebenwirkungen führt, durch spezielle individuelle anatomische Randbedingungen (z. B. im Sinne der Lage von Blutgefäßen, die bei der Implantation der Elektroden geschont werden müssen) oder auch durch sub-optimale oder gar fehlerhafte Elektrodenimplantation.

Nebenwirkungen, die durch ungünstig platzierte Elektroden verursacht werden, verschwinden nur dann, wenn die Stimulationsstärke so stark verringert wird, dass die gewünschten Wirkungen in nicht ausreichendem Maß bzw. überhaupt nicht mehr auftreten. Analog kann ein ungenügender Stimulationseffekt nicht durch ein beliebig starkes Anwachsen der Stimulationsintensität kompensiert werden, da es hierdurch typischerweise zu Nebenwirkungen oder sogar Schädigungen des Gewebes kommt.

Gegebenenfalls müssen schlecht platzierte Elektroden oder sonstige Stimulationsmittel neu implantiert werden, um sie so zu platzieren, dass die Behandlung effizient ist. Eine erneute Implantation ist immer mit einem Risiko verbunden, z. B. durch die Gefahr einer Verletzung von Gefäßen oder einer Infektion.

Herkömmliche Vorrichtungen zur Stimulation von Neuronen sind in den Dokumenten DE 10 2008 052 078 A1, DE 10 2009 015 723 A1, DE 10 2010 016 461 A1 und DE 10 2010 016 404 A1 beschrieben.

Der Erfindung liegt die Aufgabe zugrunde, eine Vorrichtung zur Stimulation von Neuronen anzugeben, mit der sich trotz verringerter Reizstärke und ohne signifikant erhöhte Stimulationsdauer robuste therapeutische Effekte erzielen lassen.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1    eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2    eine schematische Darstellung eines fraktionierten Übergangs von einem ersten Stimulationsmodus in einen zweiten Stimulationsmodus;

Fig. 3A    eine schematische Darstellung einer CR-Stimulation mit rasch variierenden Reizsequenzen;

Fig. 3B    eine schematische Darstellung einer CR-Stimulation mit langsam variierenden Reizsequenzen;

Fig. 4    eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 5    eine schematische Darstellung einer Vorrichtung zur elektrischen Stimulation von Neuronen mit einer krankhaft synchronen und oszillatorischen neuronalen Aktivität; und

Fig. 6 bis 9    Diagramme mit Simulationsergebnissen für verschiedene CR-Stimulationen.

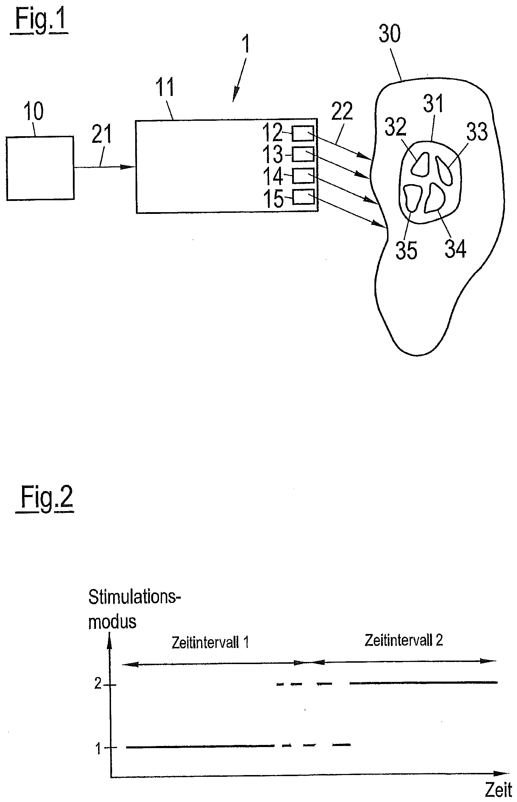

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die eine Mehrzahl von Stimulationselementen aufweist. In Fig. 1 sind beispielhaft vier Stimulationselemente 12, 13, 14 und 15 dargestellt. Die Stimulationseinheit 11 kann selbstverständlich aber auch eine andere Zahl von Stimulationselementen aufweisen, beispielsweise 2, 3, 5, 6 usw. Im Fall von elektrischer Stimulation kann es sich bei den Stimulationselementen 12 bis 15 z. B. um Stimulationskontaktflächen einer oder mehrerer Elektroden zur Applikation elektrischer Reize handeln. Falls optisch stimuliert wird, können z. B. Lichtleiter als Stimulationselemente 12 bis 15 eingesetzt werden, um das neuronale Gewebe an den gewünschten Stellen mit Lichtreizen zu stimulieren.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 wird operativ in den Körper des Patienten implantiert und erzeugt anhand der Steuersignale 21 Reize 22, insbesondere elektrische und/oder optische Reize 22, die dem Gehirn und/oder Rückenmark 30 des Patienten verabreicht werden. Die Reize 22 sind dazu ausgelegt, bei einer Verabreichung an den Patienten die krankhaft synchrone und oszillatorische neuronale Aktivität zu unterdrücken und insbesondere die Neuronen mit der krankhaft synchronen und oszillatorischen Aktivität zu desynchronisieren.

Die Steuereinheit 10 kann eine nicht-invasive Einheit sein, d. h., während des Betriebs der Vorrichtung 1 befindet sie sich außerhalb des Körpers des Patienten und wird nicht operativ in den Körper des Patienten implantiert.

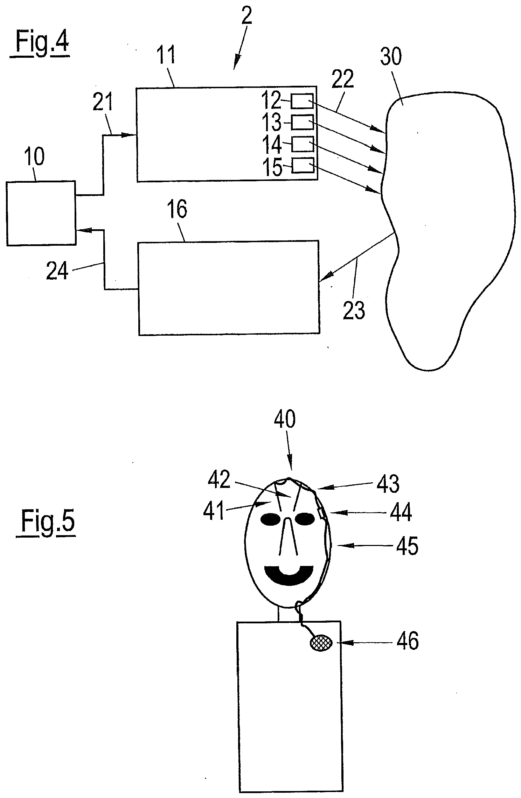

Die Vorrichtung 1 und die weiter unten, im Zusammenhang mit Fig. 4 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer CR-Stimulation dargestellt. Im Gehirn und/oder Rückenmark 30 des Patienten weist mindestens eine Neuronenpopulation 31 eine wie vorstehend beschrieben krankhaft synchrone und oszillatorische neuronale Aktivität auf. Die Stimulationseinheit 11 stimuliert die krankhaft aktive Neuronenpopulation 31 im Gehirn und/oder Rückenmark 30 mit den elektrischen und/oder optischen Reizen 22 entweder direkt oder aber die Reize 22 werden über das Nervensystem an die krankhaft aktive Neuronenpopulation 31 weitergeleitet. Die Reize 22 sind so ausgestaltet, dass die zeitversetzte (oder phasenverschobene) Stimulation mit mindestens zwei Stimulationselementen eine Desynchronisation der krankhaft synchronen Aktivität der Neuronenpopulation 31 bewirkt. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize 22 bewirken in der Neuronenpopulation 30 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 31 mittels einer gezielten Stimulation kontrolliert. Da die krankhafte Neuronenpopulation 31 über die Stimulationselemente 12 bis 15 an unterschiedlichen Stellen stimuliert wird, können die jeweiligen Phasen der neuronalen Aktivität der in Fig. 1 dargestellten Subpopulationen 32 bis 35 der krankhaften Neuronenpopulation 31 zu unterschiedlichen Zeitpunkten zurückgesetzt werden, indem die Reize 22 von den Stimulationselementen 12 bis 15 zeitversetzt (oder phasenverschoben) appliziert werden. Im Ergebnis wird dadurch die krankhafte Neuronenpopulation 31, deren Neuronen zuvor synchron und mit gleicher Frequenz und Phase aktiv waren, in mehrere Subpopulationen mit unterschiedlichen Phasen aufgespalten. Beispielsweise stimuliert das Stimulationselement 12 die Subpopulation 32, das Stimulationselement 13 stimuliert die Subpopulation 33, das Stimulationselement 14 stimuliert die Subpopulation 34 und das Stimulationselement 15 stimuliert die Subpopulation 35. Innerhalb jeder der Subpopulationen 32 bis 35 sind die Neuronen nach dem Zurücksetzen der Phase weiterhin synchron und feuern auch weiterhin mit derselben pathologischen Frequenz, aber jede der Subpopulationen 32 bis 35 weist bezüglich ihrer neuronalen Aktivität die Phase auf, die ihr durch den von dem jeweiligen Stimulationselement 32 bis 35 generierten Reiz 22 aufgezwungen wurde. Dies bedeutet, dass die neuronalen Aktivitäten der einzelnen Subpopulationen 32 bis 35 nach dem Zurücksetzen ihrer Phasen weiterhin einen in etwa sinusförmigen Verlauf mit derselben pathologischen Frequenz haben, aber unterschiedliche Phasen.

Wie vorstehend beschrieben stimulieren die Stimulationselemente 12 bis 15 mit den Reizen 22 unterschiedliche Subpopulationen. Es muss sich dabei aber nicht notwendigerweise um disjunkte, d. h. vollständig voneinander getrennte Subpopulationen handeln. Die von den Stimulationselementen 12 bis 15 stimulierten Subpopulationen können einander auch überlappen.

Bedingt durch die krankhafte Interaktion zwischen den Neuronen ist der durch die Stimulation erzeugte Zustand mit mindestens zwei Subpopulationen instabil, und die gesamte Neuronenpopulation 31 nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 31 in Subpopulationen 32 bis 35 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Bei der CR-Stimulation werden üblicherweise die Reize 22 an verschiedenen Stellen des Gehirns und/oder Rückenmarks 30 zu unterschiedlichen Zeiten im Rahmen einer sogenannten Sequenz appliziert. Innerhalb einer Sequenz wird genau einmal an jedem der unterschiedlichen Stimulationsorte gereizt, d. h., in dem vorstehenden Beispiel wird jede der Subpopulationen 32 bis 35 innerhalb einer Sequenz genau einmal gereizt. Über einen weiten Bereich der Reizstärke ist die CR-Stimulation mit langsam variierender Sequenz der CR-Stimulation mit fester Sequenz oder mit rasch, z. B. von Stimulationszyklus zu Stimulationszyklus variierender Sequenz überlegen. Im Bereich sehr geringer Reizstärken führt die CR-Stimulation zu keinen anhaltenden therapeutischen Effekten, unabhängig davon, ob und wie die Sequenz variiert wird.

Wenn die CR-Stimulation mit rasch variierender Sequenz bei sehr niedriger Reizstärke appliziert wird, kommt es unter Stimulation zu einem desynchronisierenden Effekt, der jedoch typischerweise nach dem Ende der Stimulation nicht lange anhält. Bei einer CR-Stimulation mit fester oder mit langsam variierender Sequenz kommt es bei sehr niedriger Reizstärke typischerweise zu keinerlei desynchronisierendem Effekt, weder während noch nach der Stimulation.

Der Erfindung liegt folgende überraschende Beobachtung zugrunde: Eine Epoche mit einer desynchronisierenden Stimulation mit einer rasch variierenden Sequenz hat bei einer niedrigen Reizstärke zwar für sich genommen keinen lang anhaltenden desynchronisierenden Effekt, aber in Kombination mit einer Epoche mit einer desynchronisierenden Stimulation mit langsam variierender Sequenz höherer Reizstärke bewirkt sie einen lang anhaltenden desynchronisierenden Effekt, der zumindest gleichwertig oder aber sogar signifikant besser ist als der Effekt, welcher durch zwei aufeinander folgende Epochen mit einer desynchronisierenden Stimulation (mit fester, rasch variierender oder langsam variierender Sequenz) mit der höheren Reizstärke erzielt wird.

Die Erfindung nutzt folglich eine Zwei-Stufen-Stimulation, bei der in der ersten Stufe mit rasch variierender Sequenz bei einer geringen Reizstärke stimuliert wird und in der zweiten Stufe mit langsam variierender Sequenz bei einer höheren Reizstärke stimuliert wird. Zur Realisierung der beiden Stimulationsstufen kann die Stimulationseinheit 11 in zwei verschiedenen Stimulationsmodi (oder Betriebsmodi) betrieben werden. Während zumindest 75% der Zeit eines ersten Zeitintervalls betreibt die Steuereinheit 10 die Stimulationseinheit 11 in einem ersten Stimulationsmodus. Im ersten Stimulationsmodus steuert die Steuereinheit 10 die Stimulationseinheit 11 derart an, dass die Stimulationselemente 12 bis 15 Sequenzen von Reizen 22 repetitiv erzeugen und die Reihenfolge, in welcher die Stimulationselemente 12 bis 15 innerhalb einer Sequenz die Reize 22 erzeugen, für höchstens 5 nacheinander generierte Sequenzen konstant ist und danach variiert wird, wobei die Stärke der Reize 22 im ersten Stimulationsmodus kleiner oder gleich einer vorgegebenen Reizstärke ist. Auf das erste Zeitintervall folgt ein zweites Zeitintervall. Insbesondere kann sich das zweite Zeitintervall unmittelbar dem ersten Zeitintervall, d. h. ohne eine dazwischen liegende Pause anschließen. Während mindestens 75% der Zeit des zweiten Zeitintervalls betreibt die Steuereinheit 10 die Stimulationseinheit in dem zweiten Stimulationsmodus. Im zweiten Stimulationsmodus steuert die Steuereinheit 10 die Stimulationseinheit 11 derart an, dass die Stimulationselemente 12 bis 15 Sequenzen von Reizen 22 repetitiv erzeugen und die Reihenfolge, in welcher die Stimulationselemente 12 bis 15 innerhalb einer Sequenz die Reize 22 erzeugen, für mindestens 25 nacheinander generierte Sequenzen konstant ist und danach variiert wird. Die Stärke der Reize 22 im zweiten Stimulationsmodus beträgt mindestens das 1,3-fache der vorgegebenen Reizstärke.

Die vorstehend beschriebene Stimulationsform ist hinreichend robust, so dass es für den gewünschten Stimulationserfolg ausreichend ist, wenn die Stimulationseinheit 11 für nur 75% der Zeit des ersten Zeitintervalls in dem ersten Stimulationsmodus und für nur 75% der Zeit des zweiten Zeitintervalls in dem zweiten Stimulationsmodus betrieben wird. Selbstverständlich können die Betriebszeiten des ersten und des zweiten Stimulationsmodus in dem jeweiligen Zeitintervall auch erhöht werden. Beispielsweise kann die Stimulationseinheit 11 während des vollständigen ersten Zeitintervalls im ersten Stimulationsmodus und während des vollständigen zweiten Zeitintervalls im zweiten Stimulationsmodus betrieben werden. Während Zeitspannen, in denen die Stimulationseinheit 11 während des ersten Zeitintervalls nicht im ersten Stimulationsmodus oder während des zweiten Zeitintervalls nicht im zweien Stimulationsmodus betrieben wird, kann entweder keine Stimulation oder eine Stimulation, die insbesondere anders ausgeführt sein kann als die in dieser Anmeldung beschriebene Stimulation, erfolgen.

Es kann für den Patienten vorteilhaft sein, den Wechsel vom ersten Stimulationsmodus in den zweiten Stimulationsmodus nicht abrupt, sondern fraktioniert durchzuführen, wie es beispielhaft in Fig. 2 dargestellt ist. Ein abrupter Wechsel von einer unterschwelligen Stimulationsstärke im ersten Stimulationsmodus zu einer überschwelligen Stimulationsstärke im zweiten Stimulationsmodus kann sehr unangenehm, z. B. schmerzhaft sein. Um diesen Übergang angenehmer zu gestalten, kann man Gewöhnungseffekte ausnutzen, indem man im Rahmen des Übergangs vom ersten Zeitintervall zum zweiten Zeitintervall mehrmals zwischen den beiden Stimulationsmodi hin- und herschaltet. Das Ausmaß der Nebenwirkungen, z. B. Schmerzen, hängt nicht nur von der Stimulationsstärke, sondern auch von der Dauer der Reizapplikation ab. Durch Applikation kurzer Epochen im zweiten Stimulationsmodus kann das Einsetzen der Nebenwirkungen deutlich abgeschwächt werden. Es kann sogar zu Gewöhnungseffekten kommen, so dass die Nebenwirkungen im später dauerhaft applizierten zweiten Stimulationsmodus geringer ausfallen als ohne den fraktionierten Übergang. Die Dauer zwischen dem Hin- und Herschalten zwischen dem ersten und dem zweiten Stimulationsmodus kann im Rahmen des Übergangs zeitlich variieren, z. B. zunehmen, wie dies beispielhaft in Fig. 2 gezeigt ist.

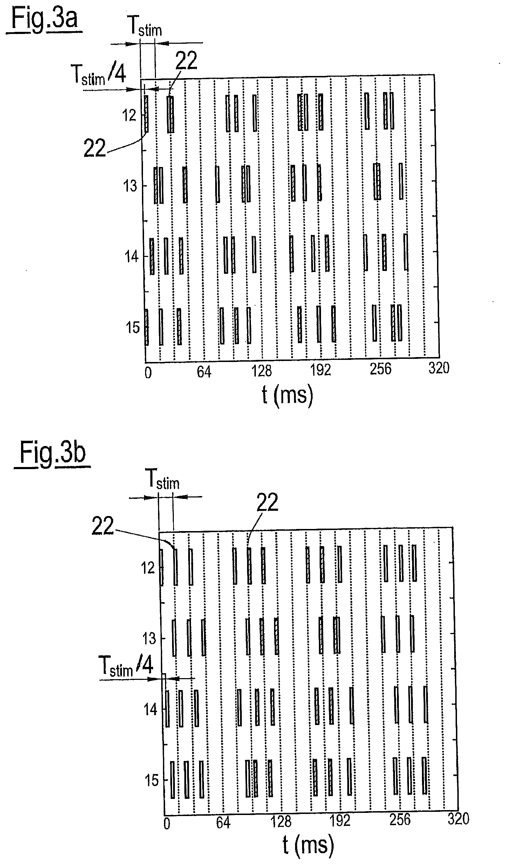

Ausführungsbeispiele für Stimulationen im ersten und zweiten Stimulationsmodus sind in Fig. 3A und 3B gezeigt.

Fig. 3A zeigt eine CR-Stimulation, bei der die vier Stimulationselemente 12 bis 15 repetitiv rasch variierende Sequenzen von Reizen 22 im ersten Stimulationsmodus erzeugen. In Fig. 3A sind untereinander die von den Stimulationselementen 12 bis 15 erzeugten Reize 22 gegen die Zeit t aufgetragen. Die Sequenzen werden in einem vorgegebenen Zeitraster, das aus aufeinanderfolgenden Zyklen besteht, erzeugt. Die einzelnen Zyklen sind in Fig. 3A durch gestrichelte Linien voneinander abgegrenzt. Jeder Zyklus weist die Länge $T_{stim}$ auf. In jedem Zyklus, in dem eine Stimulation erfolgt, erzeugen die Stimulationselemente 12 bis 15 zusammen genau eine Sequenz von Reizen 22 und jedes Stimulationselement 12 bis 15 erzeugt genau einen Reiz 22, d. h., jede Sequenz besteht in dem vorliegenden Beispiel aus einer Abfolge von vier zeitversetzten Reizen 22, die insbesondere von jeweils unterschiedlichen Stimulationselementen 12 bis 15 generiert werden, wobei sich der Zeitversatz insbesondere auf die Anfangszeitpunkte der Reize 22 beziehen kann. Zu Beginn jedes Zyklus wird in dem vorliegenden Beispiel die Reihenfolge, in welcher die Stimulationselemente 12 bis 15 die Reize 22 erzeugen, variiert. Eine unterschiedliche Füllung der in Fig. 3A gezeigten Balken, welche die Reize 22 symbolisieren, zeigt eine Variation der Reihenfolge an. Beispielsweise erzeugen die Stimulationselemente 12 bis 15 in dem ersten in Fig. 3A gezeigten Zyklus die Reize 22 in der Reihenfolge 15-12-14-13. Im zweiten Zyklus lautet die Reihenfolge 15-13-14-12 und im dritten Zyklus lautet die Reihenfolge 12-15-14-13.

Wie oben beschrieben ist vorgesehen, dass die Sequenzen für maximal 5 nacheinander generierte Sequenzen gleich bleiben und danach geändert werden. Weiterhin kann die Variation der Sequenzen mit einem konstanten Rhythmus erfolgen, d. h., eine Variation findet beispielsweise stets nach $i_{Modus\_1}$ Zyklen statt, wobei $i_{Modus\_1}$ eine ganze Zahl von 1 bis 5 ist. Alternativ kann die Anzahl der Zyklen, nach der die Sequenz variiert wird, gemäß stochastischen oder deterministischen oder gemischt stochastisch-deterministischen Regeln bestimmt werden.

Gemäß einer Ausgestaltung wird bei der in Fig. 3A gezeigten CR-Stimulation nur die Reihenfolge, in welcher die Stimulationselemente 12 bis 15 pro Sequenz die Reize 22 erzeugen, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Es kann vorgesehen sein, dass die CR-Stimulation im ersten Stimulationsmodus kontinuierlich erfolgt, d. h., in aufeinander folgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_1}$ aufeinanderfolgenden Zyklen Reize 22 erzeugt werden und während der darauffolgenden $m_{Modus\_1}$ Zyklen keine Reize 22 erzeugt werden, die dazu ausgelegt sind, die krankhaft synchrone und oszillatorische neuronale Aktivität zu desynchronisieren, wobei $n_{Modus\_1}$ und $m_{Modus\_1}$ nicht-negative ganze Zahlen sind. Das Muster aus $n_{Modus\_1}$ Zyklen mit Stimulation und $m_{Modus\_1}$ Zyklen ohne Stimulation kann periodisch fortgesetzt werden. Für die in Fig. 3A gezeigte beispielhafte Stimulationsform gilt $n_{Modus\_1} = 3$ und $m_{Modus\_1} = 2$.

Es ist denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Alternativ erzeugt die Stimulationseinheit 11 während der Stimulationspausen keinerlei Reize.

Sofern vorgesehen ist, die Sequenzen nach einer vorgegebenen Anzahl $i_{Modus\_1}$ von Sequenzen zu variieren ($i_{Moduz\_1} \leq 5$), werden gemäß einer Ausgestaltung die Zyklen ohne Stimulation nicht mitgezählt, d. h., es findet bei dieser Ausgestaltung eine Variation der Reihenfolge, in der die Stimulationselemente 12 bis 15 die Reize 22 generieren, erst dann statt, wenn tatsächlich in $i_{Modus\_1}$ Zyklen jeweils eine Sequenz von Reizen 22 appliziert wurde.

Jedes der vier Stimulationselemente 12 bis 15 stimuliert eine jeweilige der in Fig. 1 dargestellten Subpopulationen 32 bis 34 der krankhaften Neuronenpopulation 31. Während der maximal 5 Zyklen, in denen die Sequenzen konstant sind, wird von jedem der Stimulationselemente 12 bis 15 der Reiz 22 periodisch mit der Periode $T_{stim}$ appliziert. Die Reize 22 bewirken eine Phasenrücksetzung der neuronalen Aktivität der jeweils stimulierten Subpopulation. Ferner beträgt die zeitliche Verzögerung zwischen innerhalb einer Sequenz zeitlich direkt aufeinanderfolgenden, von unterschiedlichen Stimulationselementen erzeugten Reizen 22 $T_{stim}/4$, da in dem vorliegenden Ausführungsbeispiel vier Stimulationselemente 12 bis 15 für die CR-Stimulation eingesetzt werden. Für den allgemeinen Fall von P für die Stimulation verwendeten Stimulationselementen würde die zeitliche Verzögerung zwischen innerhalb einer Sequenz zeitlich direkt aufeinanderfolgenden, von unterschiedlichen Stimulationselementen erzeugten Reizen 22 $T_{stim}/P$ betragen (von diesem Wert kann auch um z. B. bis zu $\pm$ 5%, $\pm$ 10% oder $\pm$ 20% abgewichen werden). Die zeitliche Verzögerung $T_{stim}/P$ kann sich auf die Anfangszeitpunkte der Reize 22 beziehen. Die von unterschiedlichen Stimulationselementen erzeugten Reize 22 können bis auf die unterschiedlichen Startzeitpunkte identisch sein.

Die Periode $T_{stim}$, die zum einen die Dauer eines Zyklus und zum anderen die Periode angibt, mit der gleich bleibende Sequenzen sowie die von einem jeweiligen Stimulationselement 12 bis 15 generierten Reize 22 wiederholt werden, kann nahe bei der mittleren Periode der pathologischen Oszillation der Neuronenpopulation 31 mit der krankhaft synchronen und oszillatorischen neuronalen Aktivität liegen bzw. um bis zu $\pm$ 5%, $\pm$ 10% oder $\pm$ 20% von der mittleren Periode abweichen. Typischerweise liegt die Frequenz $f_{stim} = 1/T_{stim}$ im Bereich von 1 bis 30 Hz. Die Periode der pathologischen Oszillation der zu stimulierenden Neuronenpopulation 31 kann, beispielsweise mittels EEG, gemessen werden. Es ist aber auch möglich, für die Periode der pathologischen Oszillation Literatur- oder Erfahrungswerte, die sich auf die jeweilige, zu behandelnde Krankheit beziehen, zu verwenden.

Die phasenrücksetzenden Reize 22 können beispielsweise Einzelreize oder auch zusammengesetzte Reize sein. Beispielsweise kann jeder Reiz 22 aus einem Pulszug mit 2 bis 100, insbesondere 2 bis 10 Einzelpulsen bestehen. Innerhalb eines Pulszugs werden die Einzelpulse ohne Unterbrechung mit einer Frequenz im Bereich von 50 bis 500 Hz, insbesondere im Bereich von 100 bis 150 Hz, wiederholt. Die Pulse eines Pulszugs können identisch sein.

Die Stärke der Reize 22, d. h. die Amplitude der Reize 22, ist im ersten Stimulationsmodus kleiner oder gleich einer vorgegebenen Reizstärke. Die vorgegebene Reizstärke kann insbesondere unterschwellig in dem Sinne sein, dass die Reize 22 nur während der Stimulation desynchronisierende Effekte haben, die das Ende der Stimulation jedoch nicht überdauern, d. h., nach dem Ende der Stimulation mit den Reizen 22, deren Reizstärke die vorgegebene Reizstärke nicht übersteigt, verschwindet der desynchronisierende Effekt.

Die Stimulationseinheit 11 kann grundsätzlich eine beliebige Anzahl L von Stimulationselementen enthalten ($L \geq 2$), jedoch müssen bei einer Stimulation nicht zwingend alle L Stimulationselemente verwendet werden, es kann beispielsweise auch nur eine Auswahl von P der L Stimulationselemente die Reize 22 erzeugen ($2 \leq P \leq L$). Bei P Stimulationselementen ergeben sich P! mögliche unterschiedliche Sequenzen, wobei bei jeder dieser Sequenzen jedes der P Stimulationselemente genau einen Reiz 22 erzeugt. Es ist denkbar, alle P! möglichen Sequenzen für die Stimulation heranzuziehen oder aus der Menge der P! möglichen Sequenzen eine Untermenge für die Stimulation auszuwählen. Diese Untermenge kann auch in der Zeit gemäß stochastisch oder deterministisch oder gemischt stochastisch-deterministisch Regeln variieren. Die Abfolge der Sequenzen kann zufällig sein oder vor Beginn oder auch während der Stimulation festgelegt werden.

Durch die Stimulation im ersten Stimulationsmodus wird die Neuronenpopulation 31 in einen Zustand gebracht, in dem sie für die nachfolgende Stimulation im zweiten Stimulationsmodus mit langsam variierender Sequenz und höherer Reizstärke deutlich empfänglicher ist.

Die Stimulation im zweiten Stimulationsmodus kann bis auf die Anzahl der Zyklen, nach denen die Sequenz variiert wird, und die Reizstärke die gleichen Ausgestaltungen aufweisen, wie die oben im Zusammenhang mit Fig. 3A erläuterte Stimulation im ersten Stimulationsmodus. Im Folgenden werden die Unterschiede der Stimulation im zweiten Stimulationsmodus gegenüber der Stimulation im ersten Stimulationsmodus anhand von Fig. 3B erläutert.

Fig. 3B zeigt eine CR-Stimulation, bei der die vier Stimulationselemente 12 bis 15 repetitiv langsam variierende Sequenzen von Reizen 22 im zweiten Stimulationsmodus erzeugen. Die Reihenfolge, in welcher die Stimulationselemente 12 bis 15 innerhalb einer Sequenz die Reize 22 erzeugen, wird für mindestens 25 nacheinander generierte Sequenzen konstant gehalten und erst danach variiert. Es ist weiterhin denkbar, die Wiederholung derselben Sequenz zu erhöhen und die Reihenfolge, in welcher die Stimulationselemente 12 bis 15 pro Zyklus die Reize 22 erzeugen, im zweiten Stimulationsmodus für beispielsweise mindestens 30 oder mindestens 35 nacheinander generierte Sequenzen konstant zu halten. Es sei an dieser Stelle noch darauf hingewiesen, dass in Fig. 3B aus Gründen der Veranschaulichung die Sequenzen bereits nach weniger als 25 Sequenzen variiert werden. Dies ist jedoch lediglich als eine vereinfachte Darstellung einer im Vergleich zu Fig. 3A langsamen Sequenzvariation zu verstehen.

Die Variation der Sequenzen kann im zweiten Stimulationsmodus mit einem konstanten Rhythmus erfolgen, d. h., eine Variation findet beispielsweise stets nach $i_{Modus\_2}$ Zyklen statt, wobei $i_{Modus\_2} \geq 25$ gilt. Alternativ kann die Anzahl der Zyklen, nach der die Sequenz variiert wird, gemäß stochastischen oder deterministischen oder gemischt stochastisch-deterministischen Regeln bestimmt werden.

Wie bei der Stimulation im ersten Stimulationsmodus kann auch bei der Stimulation im zweiten Stimulationsmodus nur die Reihenfolge, in welcher die Stimulationselemente 12 bis 15 pro Sequenz die Reize 22 erzeugen, variiert werden. Alle übrigen Stimulationsparameter können während der Stimulation konstant bleiben.

Die Variation der Sequenzen kann z. B. stochastisch oder deterministisch oder gemischt stochastisch-deterministisch erfolgen.

Die CR-Stimulation kann im zweiten Stimulationsmodus kontinuierlich erfolgen, d. h., in aufeinander folgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_2}$ aufeinanderfolgenden Zyklen Reize 22 erzeugt werden und während der darauffolgenden $m_{Modus\_2}$ Zyklen keine Reize 22 erzeugt werden, die dazu ausgelegt sind, die krankhaft synchrone und oszillatorische neuronale Aktivität zu desynchronisieren, wobei $n_{Modus\_2}$ und $m_{Modus\_2}$ nicht-negative ganze Zahlen sind. Das Muster aus $n_{Modus\_2}$ Zyklen mit Stimulation und $m_{Modus\_2}$ Zyklen ohne Stimulation kann periodisch fortgesetzt werden. Für die in Fig. 3B gezeigte beispielhafte Stimulationsform gilt $n_{Modus\_2} = 3$ und $m_{Modus\_2} = 2$. Die Werte für $n_{Modus\_2}$ und $m_{Modus\_2}$ des zweiten Stimulationsmodus können, müssen aber nicht identisch sein mit den Werten für $n_{Modus\_1}$ bzw. $m_{Modus\_1}$ des ersten Stimulationsmodus.

Es ist denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Alternativ erzeugt die Stimulationseinheit 11 während der Stimulationspausen keinerlei Reize.

Sofern vorgesehen ist, die Sequenzen nach einer vorgegebenen Anzahl $i_{Modus\_2}$ von Sequenzen zu variieren ($i_{Modus\_2} \geq 25$), werden gemäß einer Ausgestaltung die Zyklen ohne Stimulation nicht mitgezählt, d. h., es findet bei dieser Ausgestaltung eine Variation der Reihenfolge, in der die Stimulationselemente 12 bis 15 die Reize 22 generieren, erst dann statt, wenn tatsächlich in $i_{Modus\_2}$ Zyklen jeweils eine Sequenz von Reizen 22 appliziert wurde.

Die Stärke der Reize 22, d. h. die Amplitude der Reize 22, beträgt im zweiten Stimulationsmodus mindestens das 1,3-fache der vorgegebenen Reizstärke. Die Stärke der Reize 22 kann insbesondere so groß sein, dass man einen ausgeprägten und anhaltenden therapeutischen und/oder desynchronisierenden Effekt erzielen würde, wenn man die Reize 22 während der gesamten Stimulationsdauer, d. h. während der ersten und der zweiten Zeitspanne, applizieren würde. Gemäß einer Ausgestaltung ist die untere Grenze für die Reizstärke im zweiten Stimulationsmodus größer als das 1,3-fache der vorgegebenen Reizstärke und beträgt das 1,5- oder 1,7-fache der vorgegebenen Reizstärke. Sofern es sich bei den Reizen 22 um elektrische stromgesteuerte Reize 22 handelt, ist die Reizstärke durch die Stromstärke der Reize 22 gegeben. Im Fall von optischen Reizen 22 kann die Reizstärke die Lichtstärke der Reize 22 sein.

Bei der hier beschriebenen Zwei-Stufen-Stimulation wird die Reizstärke ohne Verlust oder Einschränkung der Wirksamkeit aufdosiert. Während der ersten Stufe, d. h. im ersten Stimulationsmodus, genügt eine unterschwellige Reizstärke, wodurch ungewünschte Effekte deutlich reduziert werden können. Durch die Stimulation im ersten Stimulationsmodus wird die Neuronenpopulation 31 in einen Zustand gebracht, in dem sie für die nachfolgend in der zweiten Stufe durchgeführte Stimulation im zweiten Stimulationsmodus deutlich empfänglicher ist. Die Zwei-Stufen-Stimulation ermöglicht folglich eine verbesserte Stimulationswirkung bei gleichzeitig reduzierten Nebenwirkungen und anderen unerwünschten Effekten.

Das zugrundeliegende Wirkprinzip der Zwei-Stufen-Stimulation, nämlich die Verstärkung der desynchronisierenden Wirkung der Stimulation mit langsam variierender Sequenz durch vorgeschaltete Stimulation mit rasch variierender Sequenz, gilt nicht nur bei unterschwelliger Reizstärke der Stimulation mit rasch variierender Sequenz. Vielmehr ist die Wirkung der Zwei-Stufen-Stimulation bei überschwelliger erster Stufe zumindest tendenziell besser als alle sonstigen Varianten der CR-Stimulation gleicher Intensität und Dauer. Für den Fall der ersten Stufe mit überschwelliger Reizstärke entfällt aber der besondere Vorteil, dass durch die Verwendung der unterschwelligen Stimulation Nebenwirkungen und andere unerwünschte Effekte vermieden oder zumindest reduziert werden können.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 4 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine implantierbare Stimulationseinheit 11, welche die gleichen Funktionen und Eigenschaften wie die oben beschriebenen Steuer- und Stimulationseinheiten 10, 11 der Vorrichtung 1 aufweisen.

Darüber hinaus umfasst die Vorrichtung 2 eine Messeinheit 16. Der durch die Reize 22 erzielte Stimulationseffekt wird mit Hilfe der Messeinheit 16 überwacht. Die Messeinheit 16 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 16 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 16 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 16 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden. Derartige Befindlichkeitswerte können auch über Kurzfragebögen ermittelt werden.

Alternativ können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Tiefenelektroden sein.

Die Steuereinheit 10 verarbeitet die Signale 24, z. B. können die Signale 24 verstärkt und/oder gefiltert werden, und analysiert die verarbeiteten Signale 24. Die Steuereinheit 10 überprüft anhand der in Reaktion auf die Applikation der Reize 22 aufgenommenen Messsignale 23 den Stimulationserfolg.

Sobald insbesondere anhand der von der Messeinheit 16 aufgenommenen Messsignale 23 eine ausgeprägte Desynchronisation bzw. akute klinische Befundbesserung bzw. eine ausgeprägte Besserung der Befindlichkeit des Patienten festgestellt worden ist, kann, insbesondere mit Hilfe der Steuereinheit 10, von dem ersten Stimulationsmodus in den zweiten Stimulationsmodus umgeschaltet werden. Insbesondere kann eine mit der Steuereinheit 10 gekoppelte Eingabeeinheit vorgesehen sein, die von dem Patienten und/oder dem behandelnden Arzt bedient werden kann, und mit der vom ersten Stimulationsmodus in den zweiten Stimulationsmodus umgeschaltet werden kann.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 16, z. B. EEG-Elektroden oder Tiefelektroden (als LFP-Signal), gemessen, reicht erfahrungsgemäß die Absenkung der Synchronisation um einen vorgegebenen Wert, z. B. um mindestens 20%, im Vergleich zur Situation ohne Stimulation, um einen ausreichenden Stimulationserfolg festzustellen und vom ersten in den zweiten Stimulationsmodus zu wechseln. Es können aber größere Werte, z. B. 50% und mehr, gewählt werden, um länger im ersten Stimulationsmodus und somit mit geringerer Reizstärke zu stimulieren.

Die klinische Befundbesserung wird anhand typischer, dem Fachmann bekannter Änderungen von klinischen Scores bzw. Fragebögen bestimmt. Hierzu werden z. B. die aus der Literatur bekannten Werte Delta S für einen "minimal clinically relevant change" (minimale klinisch relevante Änderung) oder auch größere Werte, z. B. 2 x Delta S, verwandt.

Zusätzlich zu der oben beschriebenen Regelung, die das Umschalten von dem ersten in den zweiten Stimulationsmodus bestimmt, kann eine weitere Regelung vorgesehen sein, die auf einer langsameren Zeitskala agiert. Wenn sich ein Therapieerfolg über einen vordefinierten Zeitraum, z. B. 1 Stunde, eingestellt hat, wird die Stimulation abgeschaltet. Der Therapieerfolg wird hierbei wie oben beschrieben gemessen, wobei die Schwellenwerte für einen ausreichenden Therapieerfolg vom Anwender voreingestellt werden können, z. B. eine Absenkung der anfänglichen Synchronisation um 80%. Wenn diese Schwellenwerte für eine vordefinierte Dauer, z. B. 60 s, wieder überschritten werden und/oder der Patient eine nicht mehr hinreichend gebesserte Befindlichkeit meldet, wird die Zwei-Stufen-Stimulation wie oben beschrieben neu gestartet.

Mit Hilfe der Messeinheit 16 der Vorrichtung 2 können Werte für die Längen des ersten Zeitintervalls und des zweiten Zeitintervalls für einen jeweiligen Patienten abgeschätzt werden, die benötigt werden, um den gewünschten Stimulationserfolg zu erzielen. Anschließend können diese Informationen für eine Anwendung mit der Vorrichtung 1, die über keine Messeinheit verfügt, verwendet werden. Grundsätzlich können die Längen des ersten und des zweiten Zeitintervalls im Minuten- oder Stundenbereich liegen.

Weiterhin lässt sich mit Hilfe der Messeinheit 16 gemäß einer Ausgestaltung die vorgegebene Reizstärke bestimmen, aus der sich die obere bzw. untere Grenze für die Reizstärken im ersten und zweiten Stimulationsmodus ergibt. Auch diese Information kann anschließend bei einer Anwendung mit der Vorrichtung 1 genutzt werden. Zur Bestimmung der vorgegebenen Reizstärke wird die Stimulationseinheit 11 z. B. im ersten Stimulationsmodus betrieben und die Stärke der Reize 22 wird ausgehend von Null solange erhöht, bis sich ein Akuteffekt einstellt, d. h. eine Verringerung der Synchronisation der stimulierten Neuronenpopulation 31, die jedoch nach der Beendigung der Stimulation wieder verschwindet. Aus der so gewonnenen Reizstärke kann die vorgegebene Reizstärke abgeleitet werden, indem die vorgegebene Reizstärke beispielsweise aus einem Bereich ausgewählt wird, dessen untere Grenze die Reizstärke, bei der eine Verringerung der Synchronisation der stimulierten Neuronenpopulation einsetzt, darstellt und dessen obere Grenze z. B. das 1,1-fache der vorstehenden Reizstärke ist.

Die einzelnen Komponenten der Vorrichtungen 1 und 2, insbesondere die Steuereinheit 10, die Stimulationseinheit 11 und/oder die Messeinheit 16, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

Die Stimulationseinheit 11 kann z. B. ein Hirnschrittmacher sein und weist in diesem Fall eine oder mehrere implantierbare Elektroden, z. B. Tiefenelektroden, sowie gegebenenfalls dazwischen geschaltete Verbindungskabel auf. Die Elektroden der Stimulationseinheit 11 bestehen typischerweise aus einem isolierten Elektrodenschaft und einer Mehrzahl von Stimulationskontaktflächen, die in den Elektrodenschaft eingebracht worden sind.

Fig. 5 zeigt schematisch eine Vorrichtung 40 zur invasiven elektrischen Stimulation von Neuronen mit einer krankhaft synchronen und oszillatorischen neuronalen Aktivität gemäß einer Ausführungsform der Erfindung. Die Vorrichtung 40 umfasst zwei Elektroden 41, 42, die in das Gehirn des Patienten implantiert sind und über Kabel 43 mit einem Konnektor 44 verbunden sind. Der Konnektor 44 wiederum ist über ein Kabel 45 mit einer Steuereinheit 46 verbunden. Die Vorrichtung 40 kann die Funktionen der oben beschriebenen Vorrichtungen 1 und 2 aufweisen.

Implantierbare Stimulationseinheiten für die optische Stimulation von neuronalem Gewebe sind bekannt. Beispielsweise kann eine Lichtquelle, wie z. B. ein Laser, eine Laserdiode oder eine LED, einen Lichtstrahl erzeugen, der mit Hilfe einer Lichteinkopplung auf die Eingänge eines aus mehreren Lichtleitern bestehenden Faserbündels verteilt wird. Eine Steuereinheit gibt dabei z. B. vor, zu welchem Zeitpunkt ein einzelner Lichtpuls oder ein Zug von Lichtpulsen in welche Faser des Faserbündels eingekoppelt wird. Die Auskopplungspunkte der einzelnen Fasern des Fasernbündels, d. h. die Enden der Fasern, liegen an verschiedenen Stellen im Zielgebiet im Gehirn oder Rückenmark des Patienten. Das Licht stimuliert somit unterschiedliche Orte des Zielgebiets in einer durch die Steuereinheit vorgegebenen zeitlichen Abfolge. Es können allerdings auch andere implantierbare Stimulationseinheiten verwendet werden, die sich zur direkten optischen Stimulation von neuronalem Gewebe eignen.

Wie oben beschrieben bewirken die Reize 22 bei der CR-Stimulation ein Zurücksetzen, einen sogenannten Reset, der Phase der neuronalen Aktivität der stimulierten Neuronen. Mit Hilfe der von der Messeinheit 16 aufgenommenen Messsignale 23 kann die Phasenrücksetzung der einzelnen Reize 22 überprüft werden. Eine derartige Untersuchung kann vor der eigentlichen therapeutischen Zwei-Stufen-Neurostimulation vorgenommen werden.

Dazu wird über einen Sensor der Messeinheit 16 ein Signal gemessen, welches die Aktivität der über den j-ten Stimulationskanal stimulierten Subpopulation hinreichend repräsentiert. Man erhält dieses Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung zu überprüfen, besteht darin, die gemittelte Reizantwort zu bestimmen. Hierzu wird zu den Zeiten $T_1, T_2, ..., T_l$ ein Reiz mit identischen Reizparametern appliziert. Die Abstände zwischen den einzelnen Reizen $T_{k+1} - T_k$ sollten hinreichend groß und randomisiert, also nicht konstant sein, um Einschwingvorgänge zu vermeiden (vgl. P. A. Tass: Transmission of stimulus-locked responses in two coupled phase oscillators. Phys. Rev. E 69, 051909-1-24 (2004)). Typischerweise sollten die Abstände $T_{k+1} - T_k$ im Bereich von mindestens dem Zehnfachen, besser dem Hundertfachen der mittleren Periode des pathologischen Oszillation liegen. Die über alle l Test-Reize gemittelte Reizantwort wird gemäß folgender Gleichung berechnet:

$$\bar{x}_j(t) = \frac{1}{l}\sum_{k=1}^{l} x_j(\tau_k + t) \qquad\qquad (1)$$

Sofern die Abstände $T_{k+1} - T_k$ zwischen den einzelnen Reizen hinreichend groß sind, erhält man im prä-Stimulus-Bereich, d. h. im Bereich vor der Applikation eines jeweiligen Reizes, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann festgestellt werden, wenn eine gemittelte Reizantwort detektiert werden kann, d. h., wenn sich im post-Stimulus-Bereich, d. h. im Bereich für t > 0, wobei t = 0 den Anfangszeitpunkt des jeweiligen Reizes darstellt, eine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\bar{x}_j(t)$ oder $|\bar{x}_j(t)|$ betrachtet und einen charakteristischen Schwellwert, z. B. die 99te Perzentile der prä-Stimulus-Verteilung von $|\bar{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung vorliegen. D. h., die Reizstärke müsste so lange erhöht werden, bis die post-Stimulus-Antwort sich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Reize eine Phasenrücksetzung bewirken, bietet die Analyse der Phase. Hierzu wird die Phase $\psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\psi_j(t)$ kann auch mittels Wavelet-Analyse bestimmt werden.

Eine Phasenrücksetzung liegt vor, wenn die Phase $\psi_j(t)$ durch einen Reiz (mit Reiz-Beginn bei t = 0) nach einer bestimmten Zeit auf einen Vorzugswert gesetzt wird. D. h., $\{\psi_j(\tau_k+t)\}_{k=1,...,l}$, die von den l Reizantworten gewonnene Verteilung der Werte der Phase $\psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex p(t) mittels zirkulärem Mittelwert:

$$\rho(t) = \left| \frac{1}{l} \sum_{k=1}^{l} \exp\left[ i\,\psi_j(\tau_k + t) \right] \right|$$

(2)

Eine Phasenrücksetzung liegt vor, wenn p(t) z. B. das Maximum oder die 99te Perzentile der prä-Stimulus-Verteilung von p(t) (an einem Zeitpunkt oder innerhalb eines kleinen Zeitfensters von z. B. 20 ms Breite) überschreitet.

In der Praxis hat sich die Analyse mit den gemittelten Antworten $\bar{x}_j(t)$ als ausreichend bewährt.

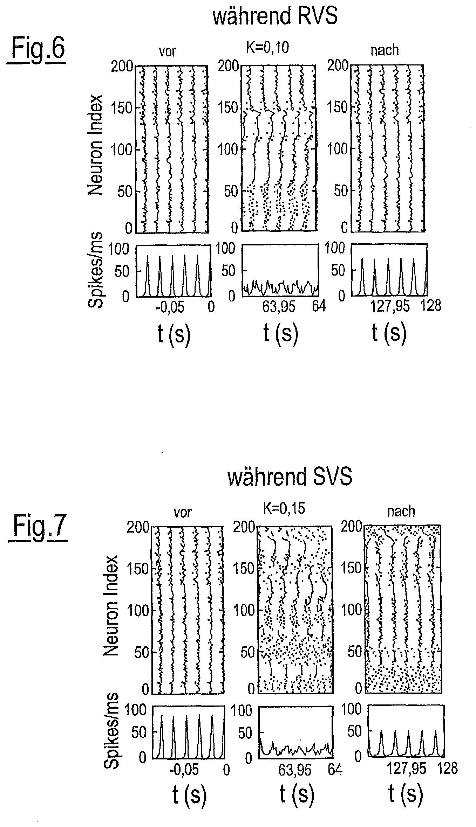

In den Fig. 6 bis 9 werden die mit der hierin beschriebenen Erfindung erzielbaren Effekte anhand von Simulationsergebnissen veranschaulicht. Der Simulation liegt ein Netzwerk von 200 Neuronen zugrunde, wobei alle Neuronen untereinander eine starke anregende kurzreichweitige Kopplung und eine schwache inhibitorisehe langreichweitige Kopplung aufweisen. Die synaptischen Kopplungsstärken in dem Netzwerk können sich gemäß STDP (Spike Timing-Dependent Plasticity)-Regeln ändern. Ein anfänglich stark gekoppeltes Netzwerk produziert hoch synchrone neuronale Aktivität.

Für die in den Fig. 6 und 7 gezeigten Simulationen wurde die jeweilige CR-Stimulation bei t = 0 s gestartet und bei t = 64 s beendet. Die Aktivität des Netzwerks wurde bis t = 128 s untersucht, d. h., bis 64 s nach Ende der Stimulation. Während der CR-Stimulation wurde kontinuierlich, d. h. ohne Zyklen, in denen Pausen eingehalten wurden, stimuliert.

In den Fig. 6 und 7 zeigen die Abbildungen in der oberen Reihe jeweils, wann jedes einzelne Neuron während verschiedener Zeitintervalle feuert: vor, während und nach Anwendung der CR-Stimulation. Die linke Spalte zeigt die neuronale Aktivität während der letzten 80 ms vor Beginn der CR-Stimulation. Die mittlere Spalte zeigt die neuronale Aktivität während der letzten 80 ms der CR-Stimulation, und die rechte Spalte bezieht sich auf die letzten 80 ms am Ende des anschließenden 64 s dauernden stimulationsfreien Zeitraums. Die Abbildungen in der oberen Reihe sind Rasterplots der neuronalen Aktivität, d. h., elektrische Entladungen der einzelnen Neuronen sind als Punkte gekennzeichnet. Die Abbildungen in der unteren Reihe zeigen jeweils, wie viele Neuronen gleichzeitig innerhalb von 1 ms während der jeweiligen Zeiträume feuern.

Für die in Fig. 6 gezeigte Simulation wurde eine CR-Stimulation mit rasch variierender Sequenz und unterschwelliger Reizstärke (RVS) verwendet. Als Reizstärke wurde K = 0,10 gewählt. Für die in Fig. 7 gezeigte Simulation wurde eine CR-Stimulation mit langsam variierender Sequenz und überschwelliger Reizstärke (SVS) verwendet, wobei die Reizstärke K = 0,15 betrug und jede Sequenz 100mal wiederholt wurde, bevor eine neue Sequenz appliziert wurde. Das Netzwerk befand sich vor der Anwendung der CR-Stimulationen in Fig. 6 und 7 im gleichen Anfangszustand.

Wie die mittlere Spalte von Fig. 6 zeigt, reicht die rasch variierende CR-Stimulation mit geringer Reizstärke für einen Akuteffekt aus, bei dem sich der Synchronisationsgrad während der CR-Stimulation verringert und nach der Beendigung der CR-Stimulation wieder signifikant erhöht. Allerdings führt auch die in Fig. 7 gezeigte alleinige Anwendung einer langsam variierenden CR-Stimulation mit höherer Reizstärke zu keiner langfristigen Desynchronisation.

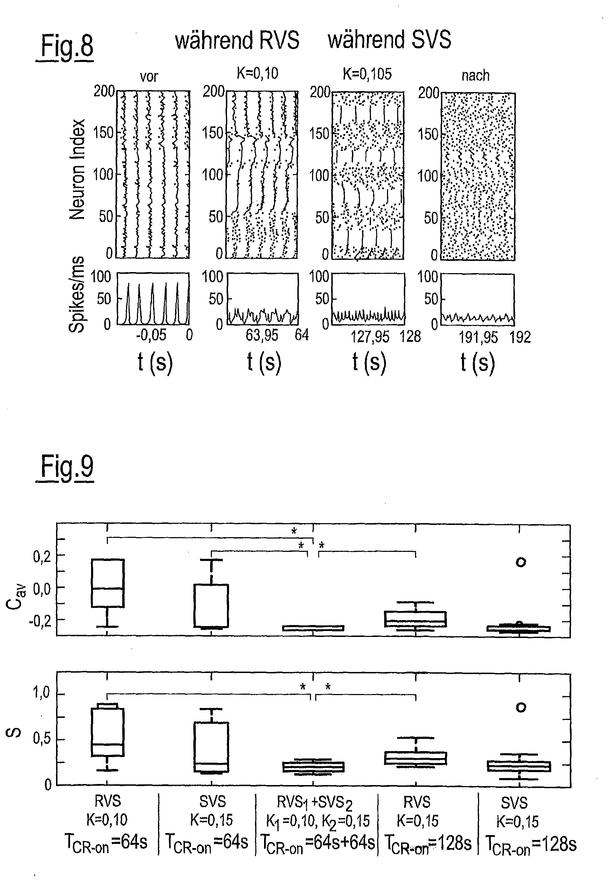

Für die in Fig. 8 gezeigte Simulation wurden die CR-Stimulationen aus den Fig. 6 und 7 miteinander kombiniert. In Fig. 8 zeigt die erste Spalte von links die neuronale Aktivität während der letzten 80 ms vor Beginn der CR-Stimulation. Die zweite Spalte zeigt die neuronale Aktivität während der letzten 80 ms der CR-Stimulation mit rasch variierender Sequenz und unterschwelliger Reizstärke. Die dritte Spalte zeigt die neuronale Aktivität während der letzten 80 ms der CR-Stimulation mit langsam variierender Sequenz und überschwelliger Reizstärke. Die vierte Spalte bezieht sich auf die letzten 80 ms am Ende des anschließenden 64 s dauernden stimulationsfreien Zeitraums. Es wurden dieselben Stimulationsparameter wie in den Fig. 6 und 7 verwendet.

Überraschenderweise zeigt sich, dass durch die nacheinander angewandten CR-Stimulationen mit rasch variierender Sequenz und mit langsam variierender Sequenz ein Stimulationserfolg erzielt wird, der auch nach 64 s nach dem Ende der CR-Stimulation noch anhält.

Da die Anfangsbedingungen des Netzwerks und die Reihenfolgen der aufeinander folgenden Sequenzen von Einfluss auf die Desynchronisation sind, wurden die Simulationen für zehn andere Kombinationen von Anfangsbedingungen des Netzwerks und Sequenzfolgen wiederholt. In Fig. 9 werden die Verteilungen $C_{av}$ der resultierenden Mittelwerte der synaptischen Stärke und die Verteilungen S der Synchronisation nach dem Beenden der CR-Stimulation bei einer Stimulationsdauer von 64 s (zwei linke Spalten) oder 128 s (drei rechte Spalten) als Boxplots für fünf verschiedene Kombinationen von CR-Methoden und Reizstärken K gezeigt. Die totale Dauer der CR-Stimulation wird als $T_{CR-on}$ bezeichnet. Die Mediane der resultierenden $C_{av}$- und S-Verteilungen werden durch die horizontalen Linien in den Boxen (Kästen) dargestellt. Die Box entspricht dem Bereich, in dem die mittleren 50% der Daten liegen, die Antennen ("whiskers") entsprechen den unteren bzw. oberen 25% der Daten. Offene Kreise bezeichnen Ausreißer und sind definiert als Werte, die sich um mindestens das 1,5-fache der Länge der Box unterhalb oder oberhalb der Box befinden. Ein Stern zeigt an, dass die Zwei-Stufen-Stimulation (mittlere Spalte) mit einer anfänglichen CR-Stimulation mit rasch variierender Sequenz und unterschwelliger Reizstärke und einer sich daran anschließenden CR-Stimulation mit langsam variierender Sequenz und überschwelliger Reizstärke statistisch signifikant erfolgreicher ist als die 64 s bzw. 128 s dauernde Ein-Stufen-Stimulation (einseitiger Mann-Whitney-U-Test, $p < 0,05$).

**Patentansprüche**

1. Vorrichtung (1; 2) zur Stimulation von Neuronen (31), umfassend

   - eine in den Körper eines Patienten implantierbare Stimulationseinheit (11) mit einer Mehrzahl von Stimulationselementen (12-15) zur Stimulation von Neuronen (31) im Gehirn und/oder Rückenmark (30) des Patienten mit Reizen (22), und
   - eine Steuereinheit (10), welche die Stimulationseinheit (11) während eines ersten Zeitintervalls und eines dem ersten Zeitintervall nachfolgenden zweiten Zeitintervalls in unterschiedlichen Stimulationsmodi betreibt, wobei
   - die Steuereinheit (10) die Stimulationseinheit (11) während mindestens 75% der Dauer des ersten Zeitintervalls in einem ersten Stimulationsmodus derart ansteuert, dass die Stimulationselemente (12-15) Sequenzen von Reizen (22) repetitiv erzeugen und die Reihenfolge, in welcher die Stimulationselemente (12-15) innerhalb einer Sequenz die Reize (22) erzeugen, für höchstens 5 nacheinander generierte Sequenzen konstant ist und danach variiert wird,
   - die Steuereinheit (10) die Stimulationseinheit (11) während mindestens 75% der Dauer des zweiten Zeitintervalls in einem zweiten Stimulationsmodus derart ansteuert, dass die Stimulationselemente (12-15) Sequenzen von Reizen (22) repetitiv erzeugen und die Reihenfolge, in welcher die Stimulationselemente (12-15) innerhalb einer Sequenz die Reize (22) erzeugen, für mindestens 25 nacheinander generierte Sequenzen konstant ist und danach variiert wird, und
   - wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

2. Vorrichtung (1; 2) nach Anspruch 1, wobei die Sequenzen im ersten und/oder zweiten Stimulationsmodus in einem Zeitraster, das aus aufeinanderfolgenden Zyklen besteht, erzeugt werden und zumindest in einigen der Zyklen jeweils eine Sequenz von Reizen (22) erzeugt wird.

3. Vorrichtung (1; 2) nach Anspruch 2, wobei innerhalb eines jeweiligen Zyklus entweder genau eine Sequenz von Reizen (22) erzeugt wird oder keine Reize erzeugt werden.

4. Vorrichtung (1; 2) nach Anspruch 2 oder 3, wobei während n aufeinanderfolgenden Zyklen Reize (22) erzeugt werden und während der darauffolgenden m Zyklen keine Reize erzeugt werden und dieses Muster periodisch fortgesetzt wird, wobei n und m nicht-negative ganze Zahlen sind.

5. Vorrichtung (1; 2) nach einem der vorhergehenden Ansprüche, wobei das Muster, nach welchem im ersten Stimulationsmodus die Reihenfolge, in welcher die Stimulationselemente (12-15) innerhalb einer Sequenz die Reize (22) erzeugen, für höchstens 5 nacheinander generierte Sequenzen konstant ist und danach die Reihenfolge variiert wird, mehrfach wiederholt wird.

6. Vorrichtung (1; 2) nach einem der vorhergehenden Ansprüche, wobei das Muster, nach welchem im zweiten Stimulationsmodus die Reihenfolge, in welcher die Stimulationselemente (12-15) innerhalb einer Sequenz die Reize (22) erzeugen, für mindestens 25 nacheinander generierte Sequenzen konstant ist und danach die Reihenfolge variiert wird, mehrfach wiederholt wird.

7. Vorrichtung (1; 2) nach einem der vorhergehenden Ansprüche, wobei die Stimulationseinheit (11) L Stimulationselemente (12-15) enthält und in einer jeweiligen Sequenz von Reizen (22) P der L Stimulationselemente (12-15) Reize (22) erzeugen, wobei $L \geq 2$ und $2 \leq P \leq L$ gilt.

8. Vorrichtung (1; 2) nach Anspruch 7, wobei jedes der P Stimulationselemente (12-15) innerhalb einer jeweiligen Sequenz von Reizen (22) genau einen Reiz (22) erzeugt.

**9.** Vorrichtung (1; 2) nach Anspruch 8, wobei genau ein Reiz (22) genau ein elektrischer und/oder optischer Pulszug ist.

**10.** Vorrichtung (2) nach einem der vorhergehenden Ansprüche, umfassend eine Messeinheit (16) zum Aufnehmen von Messsignalen (23), die eine neuronale Aktivität der mit den Reizen (22) stimulierten Neuronen (31) wiedergeben.

**11.** Vorrichtung (2) nach Anspruch 10, wobei die Steuereinheit (10) derart ausgelegt ist, dass sie von dem ersten Stimulationsmodus in den zweiten Stimulationsmodus umschaltet, wenn die Steuereinheit (10) anhand der Messsignale (23) feststellt, dass ein Synchronisationsgrad der stimulierten Neuronen (31) durch die Applikation der Reize (22) im ersten Stimulationsmodus um mindestens einen vorgegebenen Schwellwert reduziert wird.

**12.** Vorrichtung (1; 2) nach einem der vorhergehenden Ansprüche, wobei die Reize (22) elektrische und/oder optische Reize (22) sind.

**Claims**

**1.** An apparatus (1; 2) for stimulating neurons (31), comprising

- a stimulation unit (11) which is implantable into a patient's body, said stimulation unit having a plurality of stimulation elements (12-15) for stimulating neurons (31) in the patient's brain and/or spinal cord (30) with stimuli (22), and
- a control unit (10) operating the stimulation unit (11) in different stimulation modes during a first time interval and a second time interval following the first time interval, wherein
- the control unit (10) controls the stimulation unit (11) in a first stimulation mode during at least 75% of the length of the first time interval such that the stimulation elements (12-15) repetitively generate stimulus sequences (22), and that the order in which the stimulation elements (12-15) generate the stimuli (22) within a sequence remains constant for at least 5 successively generated sequences and is then being varied,
- the control unit (10) controls the stimulation unit (11) in a second stimulation mode during at least 75% of the length of the second time interval such that the stimulation elements (12-15) repetitively generate stimulus sequences (22), and that the order in which the stimulation elements (12-15) generate the stimuli (22) within a sequence remains constant for at least 25 successively generated sequences and is then being varied,
- wherein the intensity of the stimuli (22) in the first stimulation mode is smaller than or equal to a predetermined stimulus intensity and the intensity of the stimuli (22) in the second stimulation mode is at least 1.3 times higher than the predetermined stimulus intensity.

**2.** The apparatus (1; 2) according to claim 1, wherein the sequences in the first and/or second stimulation mode are generated with a time pattern consisting of successive cycles and wherein one stimulus sequence (22) is generated in at least some of the cycles.

**3.** The apparatus (1; 2) according to claim 2, wherein either exactly one stimulus sequence (22) is generated or no stimuli are generated during one respective cycle.

**4.** The apparatus (1; 2) according to claim 2 or 3, wherein stimuli (22) are generated during n successive cycles and no stimuli are generated during the subsequent m cycles, wherein this pattern is continued periodically, wherein n and m are non-negative integers.

**5.** The apparatus (1; 2) according to any one of the preceding claims, wherein the pattern, according to which the order in which the stimulation elements (12-15) generate the stimuli (22) in the first stimulation mode within a sequence remains constant for at most 5 successively generated sequences and is then being varied, is repeated for a number of times.

**6.** The apparatus (1; 2) according to any one of the preceding claims, wherein the pattern, according to which the order in which the stimulation elements (12-15) generate the stimuli (22) in the second stimulation mode within a sequence remains constant for at least 25 successively generated sequences and is then being varied, is repeated for a number of times.

**7.** The apparatus (1; 2) according to any one of the preceding claims, wherein the stimulation unit (11) contains L stimulation elements (12-15) and in a respective stimulus sequence (22) P of the L stimulation elements (12-15) generate stimuli (22), wherein $L \geq 2$, and $2 \leq P \leq L$ applies.

8.  The apparatus (1; 2) according to claim 7, wherein each of the P stimulation elements (12-15) generates exactly one stimulus (22) within a respective stimulus sequence (22).

9.  The apparatus (1; 2) according to claim 8, wherein exactly one stimulus (22) is exactly one electrical and/or optical pulse train.

10. The apparatus (2) according to any one of the preceding claims, comprising a measuring unit (16) for recording measuring signals (23) which represent a neuronal activity of the neurons (31) stimulated with the stimuli (22).

11. The apparatus (2) according to claim 10, wherein the control unit (10) is configured such that it switches from the first stimulation mode to the second stimulation mode when the control unit (10) determines, on the basis of the measuring signals (23), that a degree of synchronization of the stimulated neurons (31) is being reduced by at least one predetermined threshold value by applying the stimuli (22) in the first stimulation mode.

12. The apparatus (1; 2) according to any one of the preceding claims, wherein the stimuli (22) are electrical and/or optical stimuli (22).

## Revendications

1.  Dispositif (1 ; 2) pour la stimulation de neurones (31), comprenant

    - une unité de stimulation (11) implantable dans le corps d'un patient avec une multiplicité d'éléments de stimulation (12 - 15) pour la stimulation de neurones (31) dans le cerveau et/ou la moelle épinière (30) du patient avec des excitations (22),
    et
    - une unité de commande (10), qui actionne l'unité de stimulation (11) pendant un premier intervalle de temps et un second intervalle de temps succédant au premier intervalle de temps dans des modes de stimulation différents, où
    - l'unité de commande (10) commande l'unité de stimulation (11) pendant au moins 75 % de la durée du premier intervalle de temps dans un premier mode de stimulation de telle sorte que les éléments de stimulation (12 - 15) produisent de manière répétitive des séquences d'excitations (22) et la succession dans laquelle les éléments de stimulation (12 - 15) produisent les excitations (22) à l'intérieur d'une séquence est constante pour au plus 5 séquences générées les unes à la suite des autres puis est amenée à varier,
    - l'unité de commande (10) commande l'unité de stimulation (11) pendant au moins 75 % de la durée du second intervalle de temps dans un second mode de stimulation de telle sorte que les éléments de stimulation (12 - 15) produisent de manière répétitive des séquences d'excitations (22) et la succession dans laquelle les éléments de stimulation (12 - 15) produisent les excitations (22) à l'intérieur d'une séquence est constante pour au moins 25 séquences générées les unes à la suite des autres puis est amenée à varier, et
    - où l'intensité des excitations (22) dans le premier mode de stimulation est inférieure ou égale à une intensité d'excitation prédéterminée et l'intensité des excitations (22) dans le second mode de stimulation est au moins 1,3 fois l'intensité d'excitation prédéterminée.

2.  Dispositif (1 ; 2) selon la revendication 1, où les séquences dans le premier et/ou le second mode de stimulation sont produites dans un réseau temporel qui consiste en cycles qui se succèdent et à chaque fois une séquence d'excitations (22) est produite au moins dans quelques-uns des cycles.

3.  Dispositif (1 ; 2) selon la revendication 2, où, à l'intérieur d'un cycle respectif, exactement une séquence d'excitations (22) est produite ou aucune excitation n'est produite.

4.  Dispositif (1 ; 2) selon la revendication 2 ou 3, où pendant n cycles qui se succèdent des excitations (22) sont produites et pendant les m cycles qui suivent aucune excitation n'est produite et cette configuration est prolongée périodiquement, où n et m sont des nombres entiers non négatifs.

5.  Dispositif (1 ; 2) selon l'une des revendications précédentes, où la configuration selon laquelle dans le premier mode de stimulation la succession dans laquelle les éléments de stimulation (12 - 15) produisent les excitations (22) à l'intérieur d'une séquence est constante pour au plus 5 séquences générées les unes à la suite des autres et ensuite la succession est amenée à varier, est répétée plusieurs fois.

6. Dispositif (1 ; 2) selon l'une des revendications précédentes, où la configuration selon laquelle dans le second mode de stimulation la succession dans laquelle les éléments de stimulation (12 - 15) produisent les excitations (22) à l'intérieur d'une séquence est constante pour au moins 25 séquences générées les unes à la suite des autres et ensuite la succession est amenée à varier, est répétée plusieurs fois.

7. Dispositif (1 ; 2) selon l'une des revendications précédentes, où l'unité de stimulation (11) contient L éléments de stimulation (12 - 15) et dans une séquence d'excitations (22) respective P des L éléments de stimulation (12 - 15) produisent des excitations (22), où $L \geq 2$ et $2 \leq P \leq L$.

8. Dispositif (1 ; 2) selon la revendication 7, où chacun des P éléments de stimulation (12 - 15) produit exactement une excitation (22) à l'intérieur d'une séquence d'excitations (22) respective.

9. Dispositif (1 ; 2) selon la revendication 8, où exactement une excitation (22) est exactement un train d'impulsions électriques et/ou optiques.

10. Dispositif (2) selon l'une des revendications précédentes, comprenant une unité de mesure (16) pour la réception de signaux de mesure (23), qui traduisent une activité neuronale des neurones (31) stimulés avec les excitations (22).

11. Dispositif (2) selon la revendication 10, où l'unité de commande (10) est agencée de telle sorte qu'elle passe du premier mode de stimulation dans le second mode de stimulation quand l'unité de commande (10) établit au moyen des signaux de mesure (23) qu'un degré de synchronisation des neurones (31) stimulés est réduit par l'application des excitations (22) dans le premier mode de stimulation d'au moins une valeur seuil prédéterminée.

12. Dispositif (1 ; 2) selon l'une des revendications précédentes, où les excitations (22) sont des excitations (22) électriques et/ou optiques.

## Fig.1

## Fig.2

Fig.3a

Fig.3b

Fig.4

2

11

21

10

22

30

12
13
14
15

16

24

23

40

42
43

41
44

Fig.5

45

46

## während RVS

Fig.6

## während SVS

Fig.7

## Fig.8

während RVS   während SVS

vor   K=0,10   K=0,105   nach

## Fig.9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008052078 A1
- DE 102009015723 A1
- DE 102010016461 A1
- DE 102010016404 A1

**In der Beschreibung aufgeführte Nicht-Patentliteratur**

- **P. TEMPERLI ; J. GHIKA ; J.-G. VILLEMURE ; P. BURKHARD ; J. BOGOUSSLAVSKY ; F. VINGERHOETS.** How do parkinsonian signs return after discontinuation of subthalamic DBS?. *Neurology,* 2003, vol. 60, 78
- **P. A. TASS ; L. QIN ; C. HAUPTMANN ; S. DOVEROS ; E. BEZARD ; T. BORAUD ; W. G. MEISSNER.** Coordinated reset neuromodulation has sustained after-effects in parkinsonian monkeys. *Annals of Neurology,* 2012, vol. 72, 816-820
- **I. ADAMCHIC ; C. HAUPTMANN ; U. B. BARNIKOL ; N. PAWELCYK ; O.V. POPOVYCH ; T. BARNIKOL ; A. SILCHENKO ; J. VOLKMANN ; G. DEUSCHL ; W. MEISSNER.** Coordinated Reset Has Lasting Aftereffects in Patients with Parkinson's Disease. *Movement Disorders,* 2014, vol. 29, 1679
- **P. A. TASS.** Transmission of stimulus-locked responses in two coupled phase oscillators. *Phys. Rev.,* 2004, vol. E 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345